# EUROPÄISCHE PATENTSCHRIFT

(11) **EP 4 338 935 B1**
(45) Veröffentlichungstag und Bekanntmachung des Hinweises auf die Patenterteilung: **23.04.2025**
(21) Anmeldenummer: 22196193.1
(22) Anmeldetag: 16.09.2022
(51) Int. Cl.: B29C 57/04, B05B 11/00

(54) **FLÜSSIGKEITSSPENDER UND VERFAHREN ZUR HERSTELLUNG EINES FLÜSSIGKEITSSPENDERS**
LIQUID DISPENSER AND METHOD FOR MANUFACTURING A LIQUID DISPENSER
DISTRIBUTEUR DE LIQUIDE ET PROCÉDÉ DE FABRICATION D'UN DISTRIBUTEUR DE LIQUIDE

(43) Veröffentlichungstag der Anmeldung: 20.03.2024
(73) Patentinhaber: Aptar Radolfzell GmbH, 78315 Radolfzell (DE)
(72) Erfinder: Heinzle, Volker, 72505 Krauchenwies (DE)
(74) Vertreter: Witte, Weller und Partner Patentanwälte mbB Stuttgart

(56) Entgegenhaltungen:
- US-A1- 2007 113 841
- US-A1- 2019 366 376
- US-A1- 2022 105 524

## Beschreibung

Die Erfindung betrifft ein Verfahren zur Herstellung eines Flüssigkeitsspenders sowie einen insbesondere mittels dieses Verfahrens herstellbaren Flüssigkeitsspender.

Ein solcher Flüssigkeitsspender, der mittels des erfindungsgemäßen Verfahrens hergestellt werden kann, findet üblicherweise zur Abgabe pharmazeutischer oder auch kosmetischer Flüssigkeiten Verwendung. Er verfügt über einen Flüssigkeitsspeicher zur Lagerung der Flüssigkeit vor dem Austrag sowie über eine Austragöffnung, durch die die Flüssigkeit in einer Austragrichtung in die Umgebung abgegeben werden kann.

Die geeignete Formgebung der Austragöffnung und der die Austragöffnung umgebenden und sich insbesondere auch stromabwärts von dieser befindenden Austragstruktur hängt von der Art des Spenders und insbesondere auch von der gewünschten Applikationsform ab.

Typischerweise erfolgt die Herstellung eines Flüssigkeitsspenders zumindest bezüglich mancher oder der meisten Einzelteile mittels Kunststoffspritzguss. Dies bedeutet, dass Kunststoff in flüssiger Form in eine durch eine meist zweiteilige Gussform gebildete Kavität eingebracht wird, dort auskühlt und verfestigt und die Gussform anschließend geöffnet wird, so dass das entstandene Kunststoffbauteil entnommen werden kann. Soweit nicht besondere Maßnahmen wie die Verwendung sogenannter Schieber ergriffen werden, geht diese Form der Herstellung mit dem Erfordernis einher, dass beim Öffnen der Gussform das entstandene Kunststoffbauteil entformbar bleibt, also frei von Hinterschnitten ist, die das Trennen von einer der die Kavität bereitstellenden Gussformen hälften erschweren oder unmöglich machen. Diese Anforderung führt dazu, dass die Bauteile des Flüssigkeitsspenders und insbesondere auch jenes Kunststoffbauteils, welches von der Austragöffnung durchdrungen ist, bzgl. ihrer Formgebung limitiert sind und insbesondere eine Formgebung mit sich von einer Trennebene der Gussformhälften aus nicht nur verjüngende,sondern stellenweise aufweitende Form nur schwierig umzusetzen ist. Im Falle eines Flüssigkeitsspenders limitiert dies die Möglichkeiten der Formgebung der genannten Austragstruktur im Bereich der Austragöffnung.

US 2022/105524 A1 offenbart einen Flüssigkeitsspender, insbesondere Tröpfchenspender, bei dem das äußere Bauteil des Austragskopfes vorzugsweise im Spritzgussverfahren hergestellt ist.

Aus der US 2019/366376 A1 ist ein Verfahren zur Herstellung eines Führungselements für eine Pumpe zur Flüssigkeitsabgabe bekannt. Das Verfahren beinhaltet ein Kunststoffspritzgussverfahren zur Herstellung eines Grundkörpers und eine nachfolgende Formkorrektur durch mechanische Kraftbeaufschlagung, um die endültige Form des Elements zu erzeugen.

### AUFGABE UND LÖSUNG

Aufgabe der Erfindung ist es, ein Herstellungsverfahren betreffend einen Flüssigkeitsspender sowie einen mittels dieses Verfahrens herstellbaren Flüssigkeitsspender zur Verfügung zu stellen, wobei das Herstellungsverfahren in Hinblick auf Formgebung im Bereich der Austragöffnung kostengünstig eine hohe Flexibilität bieten soll.

Vorgeschlagen wird zu diesem Zweck ein Verfahren zur Herstellung eines Flüssigkeitsspenders mit einem Flüssigkeitsspeicher und einer Austragöffnung, durch die hindurch Flüssigkeit in einer Austragrichtung in die Umgebung abgebbar ist.

Konkret betrifft die Erfindung dabei die Herstellung eines Gehäusebauteils, welches von der Austragöffnung durchdrungen ist und welches eine die Austragöffnung bildende und/oder sich an die Austragöffnung anschließende umgebende Austragstruktur zur Beeinflussung einer Austragcharakteristik der Austragöffnung aufweist. Das Gehäusebauteil kann dabei ein Außengehäusebauteil sein, welches neben der Austragöffnung auch Kopplungsmittel wie ein Innengewinde oder eine Rastkante zur Ankopplung an einen Flüssigkeitsspender aufweist. Das Gehäusebauteil kann jedoch auch ein kleineres Gehäusebauteil sein, welches mit anderen Gehäusebauteilen zur Bildung einer Außenfläche eines Austragkopfes verbunden wird.

Die Austragstruktur des Gehäusebauteils umgibt den Abgabepfad und beeinflusst insbesondere durch ihre Formgebung stromabwärts des engsten Querschnitts des Abgabepfades die Austragcharakteristik. Insbesondere kann die Austragstruktur zum Zwecke der Tropfenbildung derart gestaltet sein, dass austretende Flüssigkeit an der Austragstruktur anhaftet, bis sie sich schwerkraftbedingt in Form eines Einzeltropfens löst.

Erfindungsgemäß wird vorgeschlagen, dass das Gehäusebauteil, welches von der Austragöffnung durchdrungen ist, hergestellt wird, indem zunächst in einem ersten Schritt ein Grundkörper durch Kunststoffspritzguss in einer Gussform hergestellt wird. Hierbei findet vorzugsweise eine Gussform Verwendung, die durch zwei Gussformhälften eine Kavität bildet, also keinerlei Schieber oder dergleichen zu Herstellung einer komplexeren Formgebung aufweist. Die Verwendung von Schiebern ist jedoch auch bei einem erfindungsgemäßen Verfahren möglich. Vorzugsweise findet sie dann jedoch nicht im Bereich der Austragstruktur statt.

Insbesondere weist der Grundkörper des Gehäusebauteil, also des Gehäusebauteils in einem Zwischenzustand nach Urformung durch Kunststoffspritzguss, eine Formgebung auf, bei der sich das Bauteil ausgehend von einer Trenngrenze der beiden Gussformhälften in beide Richtungen verjüngt und hierbei keinerlei Aufweitungen aufweist, die Hinterscheidungen bilden, welche die Entformung von der Gussform erschweren könnten.

Nach Abschluss des Kunststoffspritzgusses folgt in einem zweiten Schritt eine mechanische Kraftbeaufschlagung der bis dahin noch unfertigen Austragstruktur, die zu einer Verformung des Grundkörpers führt und damit zur Ausbildung der Austragstruktur mit ihrer endgültigen Formgebung führt.

Erfindungsgemäß ist also vorgesehen, dass die Herstellung des Gehäusebauteils mit der Austragöffnung zunächst klassisch über Kunststoffspritzguss erfolgt und anschließend eine plastische Verformung der Austragstruktur erfolgt, um hierdurch die Austragcharakteristik durch die Austragöffnung hindurch zu beeinflussen.

Das so entstandene Gehäusebauteil wird üblicherweise mit weiteren Bauteilen kombiniert, insbesondere mit einem den Flüssigkeitsspeicher bildenden Bauteilen sowie weiteren Bauteilen eines Austragkopfes, beispielsweise Ventil- oder Pumpenbauteilen, die im Kontext der Erfindung jedoch keine wesentliche Besonderheit aufweisen. Insbesondere kann der Austragkopf, der mit dem erfindungsgemäß hergestellten Gehäusebauteil mit Austragöffnung versehen ist, ein zusätzliches Außengehäuse umfassen, welches insbesondere an einem Stirnende über eine Durchbrechung verfügt, in die das als Flüssigkeitsabgabeteil ausgebildete Gehäusebauteil eingesetzt wird.

Das erfindungsgemäße Verfahren bietet vor allem zwei Vorteile:
Zum einen können Gehäusebauteile mit einer endseitigen Aufweitung und sich hieraus ergebend mit einer Hinterschneidung hergestellt werden, ohne dass hierfür eine besonders komplexe Gussform, beispielsweise mit separaten beweglichen Schiebern, erforderlich ist.

Zum anderen lassen sich unterschiedliche Austragstrukturen schaffen, ohne dass hierfür unterschiedliche Gussformen erforderlich sind. Stattdessen wird für zwei unterschiedliche Formgebungen der Austragstrukturen eine einheitliche Gussform verwendet und nach der Herstellung des Grundkörpers mittels Spritzgusses werden ausgehend von bis hierhin baugleichen Grundkörpern durch unterschiedliche Kraftbeaufschlagung der ursprünglich formgleichen Austragstrukturen unterschiedlich geformte Austragstrukturen geschaffen, die eine unterschiedliche Austragcharakteristik zeigen. Es wird demnach insbesondere vorgeschlagen, Gehäusebauteile mit unterschiedlichen Austragstrukturen ausgehend von baugleichen Grundkörpern herzustellen.

Bevorzugt ist es, dass die mechanische Kraftbeaufschlagung mittels eines Stempels erfolgt, der nach der urformenden Herstellung auf die Austragstruktur drückt. Insbesondere kann es sich um einen metallischen Stempel handeln, der Teil der Fertigungsanlage ist und automatisiert im Zuge des Herstellungsvorgangs mit definierten Kraft/Weg-Parametern auf die Austragstruktur wirkt. Vorzugsweise kann die Kraftbeaufschlagung dabei durch einen Stempel erfolgen, der in Haupterstreckungsrichtung der Austragöffnung an den Grundkörper zugestellt wird und dort von innen oder insbesondere von außen auf die Austragstruktur drückt, insbesondere entgegen der späteren Austrittsrichtung der Flüssigkeit an den Grundkörper angedrückt wird.

Der Stempel oder ein anderweitiges Element zum Zwecke der erfindungsgemäßen Kraftbeaufschlagung kann insbesondere eine sich aufweitende Form aufweisen, so dass er im Zuge der Annäherung an die Austragstruktur mit dieser in Kontakt gelangt und diese dann im Rahmen der fortgesetzten Zustellung zunehmend aufweitet. Die Formgebung der Austragstruktur des Grundkörpers vorder im zweiten Schritt stattfindenden Verformung ist vorzugsweise bereits mit einer innenkonischen Form oder ggf. auch mit einer innenzylindrischen Form versehen, welche dann im zweiten Schritt aufgeweitet wird, insbesondere mittels des genannten sich aufweitenden Stempels oder anderweitigen Kraftbeaufschlagungselements.

Vorzugsweise ist vorgesehen, dass an einem distalen Ende der Austragstruktur, die im zweiten Schritt erfolgende Aufweitung den Querschnitt eines lichten Innendurchmessers zumindest um 10% aufweitet, vorzugsweise um mindestens 20%.

Ziel der Kraftbeaufschlagung ist eine dauerhafte Veränderung der Austragstruktur, also eine plastische Verformung des Kunststoffmaterials. Um dies zu erzielen, erfolgt die Kraftbeaufschlagung vorzugsweise mit einem erwärmten Bauteil. Je nach verwendetem Kunststoff liegt die Temperatur des Bauteils während der Verformung vorzugsweise mindestens bei der Vicat-Erweichungstemperatur. Für die meisten Kunststoffmaterialien liegt diese Temperatur zwischen 60°C und 160°C. Das Verfahren ist grundsätzlich mit allen bei Flüssigkeitsspendern üblichen Kunststoffen verwendbar.

Die Kraftbeaufschlagung bei erwärmtem Bauteil kann insbesondere erzielt werden, indem die Kraftbeaufschlagung der Austragstruktur mittels des Stempels bei nach dem Spritzguss noch warmem Grundbauteil erfolgt. Alternativ oder zusätzlich kann auch vorgesehen sein, dass die Kraftbeaufschlagung unmittelbar mit einem erwärmten Stempel erfolgt. Insbesondere kann der Stempel zu diesem Zweck mit einem Heizelement versehen sein.

Neben der Nutzung der verbleibenden Wärme nach dem Spritzguss und der Verwendung eines erwärmten Stempels ist es natürlich auch möglich, das Grundbauteil als Ganzes in einem separaten Heizschritt nach dem Spritzguss zu erwärmen, um dann im erwärmten Zustand die Kraftbeaufschlagung stattfinden zu lassen.

Die Erwärmung der Austragstruktur während der Kraftbeaufschlagung, insbesondere die Verwendung eines erwärmten Stempels zum Zwecke der Kraftbeaufschlagung, bietet auch den Vorteil, dass hierdurch vom Spritzguss verbleibende Grate im Bereich der Austragöffnung vermindert oder beseitigt werden. Insbesondere bei der Herstellung eines Augentropfenspenders ist dies ein wesentlicher Vorteil.

Die Kraftbeaufschlagung der Austragstruktur und die damit einhergehende Verformung muss nicht zwingend mittels eines separaten Werkzeugs wie dem genannten Stempel stattfinden, sondern kann auch durch ein Element des Flüssigkeitsspenders selbst erfolgen. Insbesondere kann vorgesehen sein, dass die mechanische Kraftbeaufschlagung und die damit einhergehende Verformung mittels einer Schutzkappe des Flüssigkeitsspenders erfolgt. Die Schutzkappe weist hierzu an ihrer Innenseite eine sich von der außen in Richtung der Austragöffnung verjüngende Aufweitungsstruktur auf, die derart an die vorläufige Formgebung der Austragstruktur des Grundkörpers nach der urformenden Herstellung angepasst ist, dass diese Austragstruktur kelchartig aufgeweitet wird.

Ein solches Vorgehen erleichtert das Verfahren, da der zweite Schritt, die Verformung der Austragstruktur, mit dem Schritt der Montage der Kappe einhergeht. Vorzugsweise ist die Schutzkappe eine vom Gehäusebauteil getrenntes zweites Bauteil, welches insbesondere vorzugsweise separat hergestellt wird. Nach urformender Herstellung des Gehäusebauteils mit der Austragstruktur und gegebenenfalls Zusammenfügung dieses Gehäusebauteils mit anderen Gehäusebauteilen wie beispielsweise einem Flüssigkeitsspeicher oder Ventil- oder Pumpenbauteilen wird anschließend die Schutzkappe aufgesetzt. Während des Aufsetzens der Schutzkappe kommt es zur Kraftbeaufschlagung der Austragstruktur und insbesondere zu deren radialer Aufweitung. Insbesondere vorzugsweise ist die Austragstruktur zu diesem Zeitpunkt erwärmt, um mittels der Schutzkappe und der an dieser vorgesehenen Aufweitungsstruktur die gewünschte plastische Verformung der Austragstruktur zu erzielen. Durch die Verwendung der Schutzkappe als die Austragstruktur beeinflussendes Element können im Übrigen baugleiche Spender daher durch unterschiedliche Schutzkappen mit unterschiedlichen Austragstrukturen versehen werden.

Die Erfindung betrifft neben dem genannten Herstellungsverfahren auch einen Flüssigkeitsspender, der insbesondere gemäß diesem Verfahren hergestellt worden sein kann. Insbesondere vorzugsweise handelt es sich um einen Tropfenspender zur Abgabe diskreter Einzeltropfen. Ein solcher Tropfenspender verfügt über eine Austragstruktur, die im Bereich der Austragöffnung eine Tropfenbildungsfläche aufweist, an der sich die ausgetragene Flüssigkeit anlagert. Erst wenn die Flüssigkeitsmenge eines Tropfens erreicht ist, löst sich dieser Tropfen schwerkraftbedingt von der Austragstruktur.

Ein erfindungsgemäßer Flüssigkeitsspender verfügt über einen Flüssigkeitsspeicher sowie über eine Austragöffnung, durch die die Flüssigkeit in einer Austragrichtung in die Umgebung abgegeben werden kann. Die Austragöffnung ist vorzugsweise an einem Austragkopf vorgesehen, der zur Ankopplung an einen Flüssigkeitsspender ausgebildet ist. Der Flüssigkeitsspender kann insbesondere als Pumpspender oder als Quetschflaschenspender ausgebildet sein. Im Falle der Gestaltung als Pumpspender verfügt der Spender über eine Pumpeinrichtung mit einer Pumpkammer, die eingangsseitig und ausgangsseitig mit einem Ventil versehen ist und die mittels einer Betätigungshandhabe, insbesondere mittels eines seitlich an einem Gehäuse des Austragkopfes vorgesehenen Knopfes, betätigt werden kann, um Flüssigkeit aus der Pumpkammer auszutragen und neue Flüssigkeit aus dem Flüssigkeitsspender anzusaugen. Im Falle eines Quetschflaschenspenders ist keine Pumpeinrichtung vorgesehen. Stattdessen erfolgt die Druckbeaufschlagung dadurch, dass der Flüssigkeitsspender als Ganzes elastisch zusammengedrückt wird und die Flüssigkeit hierdurch zur Austragöffnung gedrückt wird. Sowohl im Falle eines Pumpspenders als auch im Falle eines Quetschflaschenspenders ist vorzugsweise vorgesehen, dass der Austragöffnung unmittelbar ein Austragventil vorgeschaltet ist, welches bei Überdruck öffnet und so den Ausdruck gestattet.

Der Flüssigkeitsspender weist ein Gehäusebauteil auf, insbesondere ein Außengehäuse eines Austragkopfes oder ein hierein eingesetztes Flüssigkeitsabgabeteil, welches von der Austragöffnung durchdrungen ist und welches eine die Austragöffnung bildende und/oder sich an die Austragöffnung anschließende Austragstruktur aufweist. Diese Austragstruktur, die insbesondere die oben genannte Tropfenbildungsfläche bildet und stromabwärts der Austragöffnung vorgesehen sein kann, dient der Beeinflussung der Austragcharakteristik. Die genannte Tropfenbildung ist hierfür ein Beispiel. Die Austragstruktur könnte jedoch auch eine anderweitige Formgebung aufweisen, beispielsweise um einen Flüssigkeitsstrahl zu erzeugen.

Die Besonderheit des erfindungsgemäßen Flüssigkeitsspenders liegt darin, dass die Austragstruktur an ihrer Außenseite eine umlaufende Vertiefung aufweist. Gegenüber einem Querschnitt der Austragstruktur an einem stromabwärtigen distalen Ende ist der Querschnitt der Austragstruktur im Bereich der umlaufenden Vertiefung in Art einer Einschnürung verringert. Im Falle eines Tropfenspenders ist das distale Ende oberhalb der vorzugsweise mit einem Außendurchmesser von mindestens 1 mm und/oder von maximal 3 mm ausgebildet, vorzugsweise mit einem Außendurchmesser von mindestens 1,5 mm und/oder von höchstens 2,5 mm. Im Bereich der darunterliegenden umlaufenden Vertiefung ist der Außendurchmesser vorzugsweise mindestens um 10% geringer, vorzugsweise um mindestens 20% geringer.

Wie groß der Durchmesser am distalen Ende im Einzelnen zum Zwecke der Tropfenabgabe gewählt werden sollte, hängt vom gewünschten Tropfenvolumen unter Berücksichtigung der Flüssigkeit und ihrer Viskosität und Dichte ab sowie von den Eigenschaften des Materials der Austragstruktur. Der Durchmesser sollte so gewählt werden, dass sich hier ein Tropfen des gewünschten Volumens bilden kann und sich bei Erreichen dieses Volumens zuverlässig schwerkraftbedingt von der Austragstruktur und der hier vorzugsweise vorgesehenen Kelchform löst. Das erfindungsgemäße Verfahren gestattet es, ausgehend von formgleichen Grundbauteilen nach dem Spritzgießen durch die anschließende Verformung und insbesondere Aufweitung des distalen Endes des Austragstruktur Austragstrukturen von jeweils an die Flüssigkeit ideal angepasster Formgebung herzustellen.

Die Kelchform, die sich durch die umlaufende Vertiefung unterhalb des distalen Endes der Austragstruktur ergibt, führt am oberen Rand des Kelches zu vorteilhaften geometrischen Verhältnissen, um während der Tropfenbildung das Entweichen der Flüssigkeit aus dem Inneren der Austragstruktur zu vermeiden. An der Außenseite der Austragstruktur weist diese vorzugsweise einen sich in Austragrichtung stetig aufweitenden außenseitigen Aufweitungsbereich auf, der sich von der umlaufenden Vertiefung bis zum distalen Ende der Austragstruktur erstreckt. Ein Winkel zwischen einer Oberfläche des außenseitigen Aufweitungsbereichs, also einer Geraden, die in der Ebene des Aufweitungsbereichs liegt, und der Abgaberichtung beträgt vorzugsweise mehr als 20°, insbesondere vorzugsweise mehr als 30° oder 40°.

Vorzugsweise ist die plane oder innenkonische Tropfenbildungsfläche, die von der Austragstruktur gebildet wird, außenseitig am distalen Ende der Austragstruktur von einer Abrisskante begrenzt, die ein Entweichen der Flüssigkeit erschwert. Insbesondere kann die Abrisskante mit einem Krümmungsradius von mehr als 0,05 mm, insbesondere von mehr als 0,1 mm ausgebildet sein, um Verletzungen durch die Abrisskante zu vermeiden.

Vorzugsweise ist die Austragstruktur im Bereich der Vertiefung gratfrei. Ein Grat im Bereich der Austragstruktur ist vermeidenswert, da er gerade bei Medikamenten, die in die Augen appliziert werden, eine Gefahr darstellt. Gelangt ein scharfkantiger Grat mit dem Auge in Kontakt, kann er Verletzungen verursachen.

Die Gratfreifreiheit im Bereich der Vertiefung ist insbesondere durch das oben beschriebene Verfahren erzielbar. Während eine Formgebung einer Austragstruktur mit umlaufender Vertiefung mit bislang üblicher Technik nur dadurch erzielbar ist, dass die Trennlinie der Gussformhälften im Bereich der Vertiefung vorgesehen ist oder dass eine Gussform mit Schiebern genutzt wird, gestattet es das erfindungsgemäße Verfahren, die umlaufende Vertiefung mittels der beschriebenen Zweistufigkeit zu erzeugen, also indem in einem ersten Spritzguss-Schritt die Grundform ohne Vertiefung hergestellt wird und diese anschließend durch Aufweitung der Austragstruktur oberhalb der Vertiefung gebildet wird.

Die Austragstruktur weist vorzugsweise in jeweiligen Aufweitungsbereichen außenseitig und innenseitig eine kelchartige Aufweitung auf. Vorzugsweise ist dabei ein Winkel zwischen einer Oberfläche des außenseitigen Aufweitungsbereichs und der Abgaberichtung kleiner als ein Winkel zwischen einer Oberfläche des innenseitigen Aufweitungsbereichs und der Abgaberichtung. Die kelchförmige Wandung der Austragstruktur wird zum distalen Ende hin vorzugsweise dünner.

Wie oben bereits beschrieben, ist es möglich, das erfindungsgemäße Herstellungsverfahren derart zu verwenden, dass die plastische Verformung der Austragstruktur ausgehend von der Grundform unter Nutzung der Schutzkappe als kraftbeaufschlagendes Element erfolgt.

Ein erfindungsgemäßer Flüssigkeitsspender weist daher vorzugsweise eine abnehmbare und wieder aufsetzbare Schutzkappe auf, die die Austragöffnung im aufgesetzten Zustand überdeckt und an deren Innenseite eine Aufweitungsstruktur vorgesehen ist, die bei aufgesetzter Schutzkappe an einer Innenseite der Austragstruktur anliegt. Dadurch, dass die Formgebung der Austragstruktur im Zuge der Herstellung mittels Aufweitungsstruktur bestimmt wird, liegen die Aufweitungsstruktur und die Innenseite der Austragstruktur bei aufgesetzter Schutzkappe besonders bündig aneinander an. Dies stellt auch deshalb einen Vorteil dar, da hierdurch im späteren Gebrauch Flüssigkeitsreste sehr zuverlässig aus der Austragstruktur verdrängt werden können, wenn die Schutzkappe nach Nutzung des Flüssigkeitsspenders wieder aufgesetzt wird.

Es kann von Vorteil sein, wenn das von der Austragöffnung durchdrungene Gehäusebauteil und die Schutzkappe oder zumindest deren Aufweitungsstruktur aus unterschiedlichen Kunststoffmaterialen bestehen. Insbesondere kann die Aufweitungsstruktur aus einem härteren Material und/oder einem Material mit höherer Erweichungstemperatur bestehen.

Die beim initialen Aufsetzen der Schutzkappe oder bei Verwendung des Stempels bestimmungsgemäß stattfindende Verformung der Austragstruktur findet zumindest teilweise plastisch statt, so dass eine bleibende Verformung besteht. Es kann jedoch auch vorgesehen sein und durch geeignete Verfahrensparameter, insbesondere eine geeignet ausgewählte Temperatur, erreicht werden, dass die Verformung teilweise elastisch ist, so dass bei aufgesetzter Schutzkappe die Austragstruktur unter elastischer Spannung steht.

Die Schutzkappe sollte vorzugsweise keine reine Steckkappe sein, sondern formschlüssig einer Abnahme entgegenwirken. Dies kann insbesondere durch eine Rastkante an der Schutzkappe oder die Gestaltung der Schutzkappe mit einem Gewinde zur Ankopplung an ein Außengehäuse des Austragkopfes erreicht werden.

Die Austragöffnung und die Austragstruktur des Flüssigkeitsspenders weisen üblicherweise eine rotationssymmetrische Formgebung auf. Dies ist jedoch nicht zwingend. Es kann auch von Vorteil sein, eine nicht-rotationssymmetrische Form vorzusehen, beispielsweise einen polygonalen Querschnitt oder einen Querschnitt mit umfänglich vorgesehenen diskreten Ausbuchtungen vorzusehen. Im Falle eines Tropfenspenders kann hierdurch die Trennneigung eines Tropfens beeinflusst werden.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt einen erfindungsgemäßen Flüssigkeitsspender in einer Schnittdarstellung.
Fig. 2 zeigt den Bereich der Austragöffnung des Flüssigkeitsspenders in einer vergrößerten Ansicht.
Fig. 3 und 4 zeigen die Verwendung des Flüssigkeitsspenders in unterschiedlichen Ausrichtungen.
Fig. 5A bis 5F zeigen das Verfahren zur Herstellung eines Flüssigkeitsabgabeteils 14 des Spenders 10.
Fig. 6 zeigt einen Flüssigkeitsspender, der mittels eines partiell alternativen Verfahrens hergestellt ist.

### DETAILLIERTE BESCHREIBUNG DER AUSFÜHRUNGSBEISPIELE

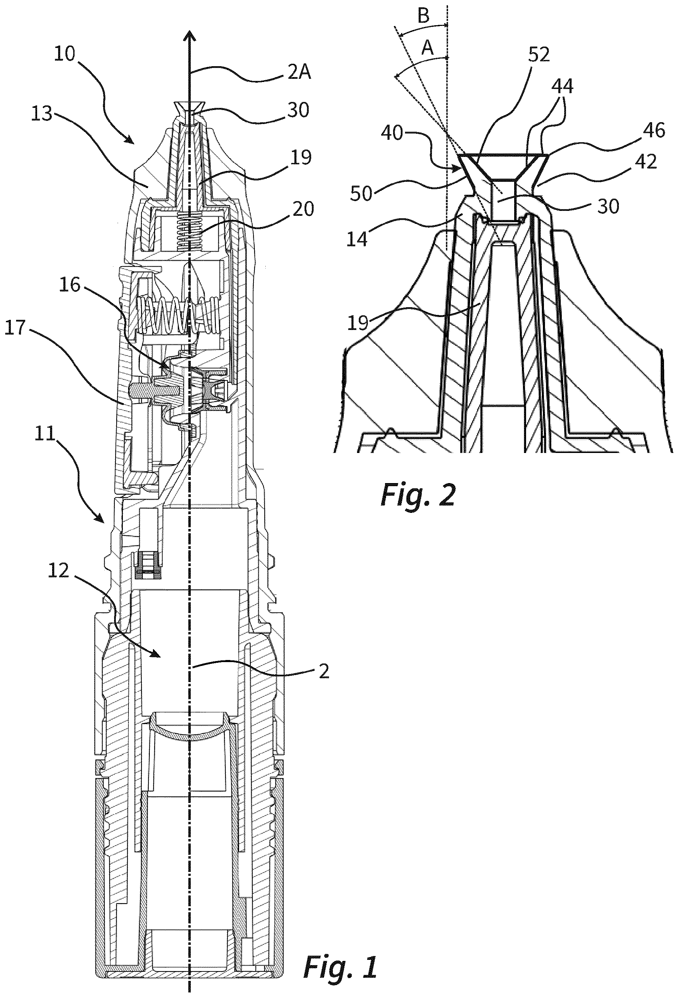

Fig. 1 zeigt einen länglich in Richtung einer Haupterstreckungsachse 2 erstreckten Flüssigkeitsspender 10, vorliegend exemplarisch einen Tropfenspender. Der Flüssigkeitsspender 10 verfügt über einen Austragkopf 11, an den mittels einer Rastverbindung ein Flüssigkeitsspeicher 12 angekoppelt ist. Innerhalb des Austragkopfes 11 ist eine Pumpeinrichtung 16 vorgesehen, die über einen seitlich am Außengehäuse 13 des Austragkopfes 11 vorgesehenen Betätigungsdrücker 17 betätigt werden kann und die bei Betätigung Flüssigkeit aus dem Flüssigkeitsspeicher 12 ansaugt und in Richtung einer Austragöffnung 30 fördert. Fig. 2 zeigt den Bereich der Austragöffnung 30 in vergrößerter Darstellung.

Die Austragöffnung 30 ist Teil eines Flüssigkeitsabgabeteils 14. Dieses ist an einem Innenbauteil 18 des Spenders 10 angebracht und begrenzt den Flüssigkeitspfad zur Austragöffnung 30. Es verfügt über einen hülsenförmigen Abschnitt, der durch eine Durchbrechung des Außengehäuse 13 hindurchragt und an dessen Ende eine im Weiteren noch erläuterte Austragstruktur 40 vorgesehen ist.

Innerhalb des Flüssigkeitsabgabeteils 14 ist ein Ventilkörper 19 vorgesehen, der von einer Schraubenfeder 20 in Richtung der Austragöffnung 30 gedrückt wird und die Austragöffnung 30 in einem Schließzustand verschließt. Erst wenn der Flüssigkeitsdruck in einem Bereich zwischen der Pumpeinrichtung 16 und der Austragöffnung 30 ein ausreichendes Druckniveau erreicht hat, öffnet das Auslassventil durch Verlagerung des Ventilkörpers 19 entgegen der Federkraft der Schraubenfeder 20 und Flüssigkeit kann austreten.

Aus Fig. 2 ist die Austagstruktur 40 im Bereich der Austragöffnung 30 gut zu erkennen. Unter der Austragstruktur 40 wird die Geometrie, der die Austragöffnung 30 umgebenden bzw. sich hieran anschließenden Bereiche des Flüssigkeitsabgabeteils 14 verstanden, die die Form der Abgabe beeinflussen.

Vorliegend handelt es sich um eine Austragstruktur 40, die in Hinblick auf die Abgabe von Tropfen gestaltet wird. Die Austragstruktur ist rotationsymmetrisch zur Haupterstreckungsachse 2 ausgebildet und verfügt über eine im Wesentlichen kelchartige Formgebung, die primär durch einen innenseitigen sich in Richtung der Austragrichtung 2A aufweitenden Aufweitungsbereich 52 und einen außenseitigen sich ebenfalls in Richtung der Austragrichtung 2A aufweitenden Aufweitungsbereich 50 begrenzt ist.

Außenseitig ist eine umlaufende Vertiefung 42 vorgesehen. Es handelt sich um eine Art Einschnürung, von der aus sich das Flüssigkeitsabgabeteil 14 in beide Richtungen aufweitet.

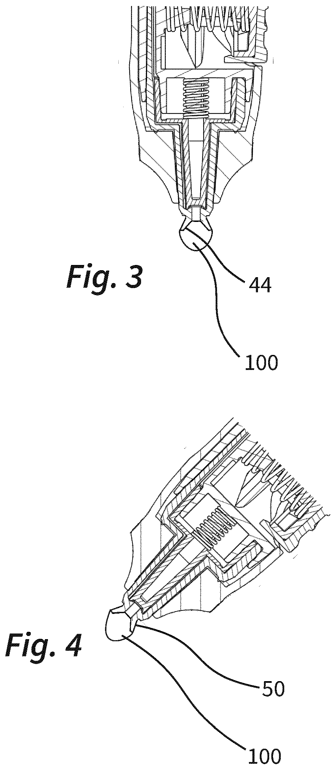

Wie Fig. 3 verdeutlicht, bildet der innere Aufweitungsbereich 52 eine Tropfenbildungsfläche 44 Flüssigkeit, die durch die Austragöffnung 30 ausgetragen wird, lagert sich zunächst im Bereich dieses Aufweitungsbereichs 52 an, bis der so gebildete Tropfen eine Masse erreicht hat, die ihn schwerkraftbedingt von der Austragstruktur 40 trennt. Der Aufweitungsbereich 52 und die Stirnfläche der Austragstruktur 40 bilden gemeinsam eine Tropfenbildungsfläche 44.

Die umlaufende Vertiefung 42 gestattet es, den dargestellten Winkel B an der Außenseite des distalen Endes des Flüssigkeitsabgabeteils 14 bereitzustellen. Dieser Winkel B, der vorzugsweise kleiner ist als der Winkel A, der zwischen der Haupterstreckungsachse 2 und dem inneren Aufweitungsbereich 52 vorgesehen ist, ist von Vorteil, um zu verhindern, dass die Flüssigkeit sich nicht nur im Aufweitungsbereich 52 und an der Stirnseite sammelt, sondern um die distale Außenkante herum auch in den außenliegenden Aufweitungsbereich 50 gelangt. Somit wird gewährleistet, dass die Flüssigkeitsmenge eines Tropfens 100 von der beabsichtigten Flüssigkeitsmenge abweicht.

Fig. 4 verdeutlicht, dass der Winkel A eine noch größere Rolle spielt, wenn der Tropfenspender im Zuge der Tropfenabgabe schräg gehalten wird. Ist dies der Fall, so steigt die Neigung der Flüssigkeit, von der inneren Tropfenbildungsfläche 44 auf den äußeren Aufweitungsbereich 50 zu gelangen. Je größer der WinkelA ist, desto unkritischer ist eine solche Schrägstellung in Hinblick auf die Erzielung eines einheitlichen Tropfenvolumens.

Die beschriebene Formgebung der Austragstruktur 40 ist per Kunststoffspritzguss nicht ohne weiteres erzielbar.

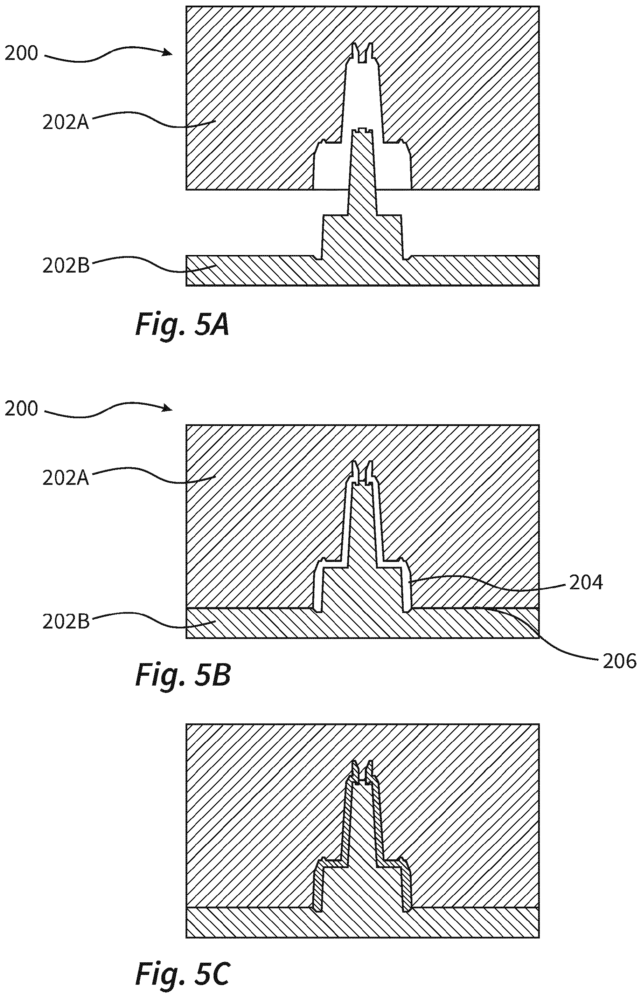

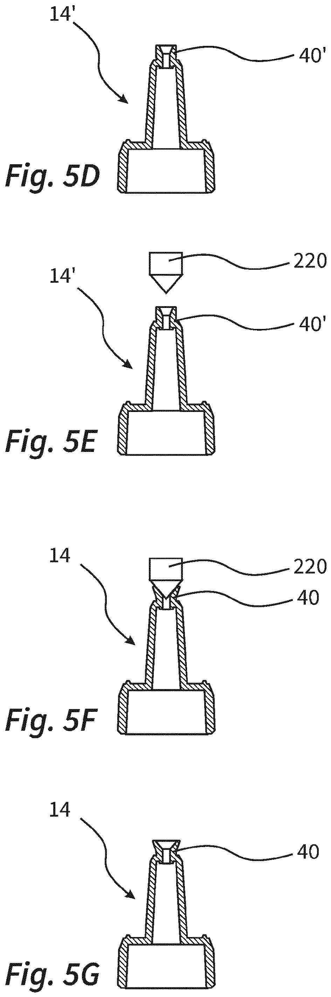

Fig. 5A bis 5F zeigen einen möglichen Ablauf zur Herstellung des Flüssigkeitsabgabeteils 14.

Zunächst wird ein Grundkörper 14' hergestellt. Dies erfolgt mittels Kunststoffspritzguss. Es findet ein Gusswerkzeug 200 mit zwei Gussformhälften 202A und 202B Verwendung, wie in Fig. 5A gezeigt. Die beiden Gussformhälften 202A, 202B bilden im geschlossenen Zustand der Fig. 5B eine Kavität 204, die die Negativform des Grundkörpers 14' aufweist. In diese Kavität 204 wird flüssiger Kunststoff eingespritzt, wie in Fig. 5C gezeigt ist.

Nach Verfestigung des Kunststoffs wird das Gusswerkzeug 200 geöffnet und der Grundkörper 14' kann entnommen werden. Wie sich anhand der Fig. 5A ersehen lässt, sind beide Gussformhälften 202A, 202B so geformt, dass sich keinerlei Hinterschneidungen ergeben, die einer Entformung des Grundkörpers 14' entgegenstehen könnten. Dieser kann daher ohne Verformung entnommen werden.

Fig. 5D zeigt den Grundkörper 14' nach dem Spritzguss. Da die Trenngrenze 206 sich abseits der im Zustand der Fig. 5D noch vorläufigen Austragstruktur 40' befindet, ist diese frei von Graten.

Ausgehend vom Zustand der Fig. 5D folgt ein zweiter Schritt der Fertigung des Flüssigkeitsabgabeteils 14. Es wird nun ein Stempel 220, der über eine konische Spitze verfügt, von der Außenseite aus und in Richtung der Haupterstreckungsachse 2 zugestellt, wie in Fig. 5E dargestellt.

Wie in Fig. 5F dargestellt, drückt dieser Stempel 220 die vorläufige Austragstruktur 40' nach außen und verformt diese dabei plastisch. Insbesondere kann zu diesem Zeitpunkt der Grundkörper 14' erwärmt sein, entweder noch aufgrund des vorausgehenden Spritzgussschritts oder aufgrund einer separaten nachfolgenden Erwärmung. Hierdurch lässt sich die Neigung der Austragstruktur 40 erhöhen, sich plastisch verformen zu lassen. Zusätzlich oder alternativ zu einer vorherigen Erwärmung des Grundkörpers 14' ist es auch denkbar, den Stempel 220 selbst zu erwärmen.

Die hierbei jeweils zu wählende Temperatur hängt vom verwendeten Kunststoff ab. Vorzugsweise liegt die Temperatur oberhalb der Erweichungstemperatur des jeweiligen Kunststoffs. Im Falle von Polypropylen liegt diese Temperatur beispielsweise bei etwa 150°C.

Sobald die plastische Verformung stattgefunden hat, kann der Stempel 220 wieder entfernt werden. Es verbleibt das nunmehr fertiggestellte Flüssigkeitsabgabeteil 14, dessen Austragstruktur 40 die bereits in Fig. 2 dargestellte Form aufweist. Durch die kelchartige Aufweitung ist eine besonders genaue Dosierung möglich, da abgegebene Tropfen bezüglich ihres Flüssigkeitsvolumens nur in sehr geringem Maße variieren.

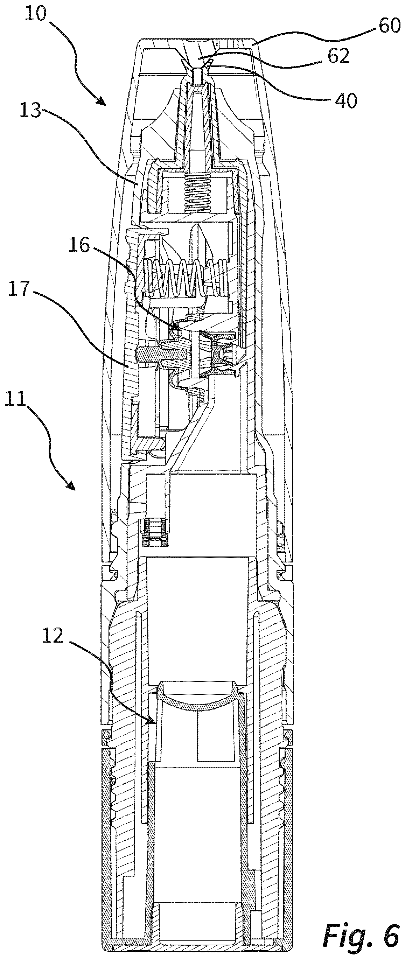

Fig. 6 zeigt eine alternative Gestaltung. Hier liegt die Besonderheit darin, dass das Flüssigkeitsabgabeteil 14 zunächst noch in Form des Grundkörpers 14' belassen wird und die plastische Verformung durch eine Schutzkappe 60 bewirkt wird.

Die Schutzkappe 60 weist zu diesem Zwecke an ihrer Innenseite eine Aufweitungsstruktur 62 auf. Vorzugsweise ist die Schutzkappe als Ganzes oder zumindest die Aufweitungsstruktur 62 aus einem härteren Material oder aus einem Material mit höherer Erweichungstemperatur verglichen mit dem Material des Flüssigkeitsabgabeteils 14 hergestellt.

Wenn die Schutzkappe 60 erstmals auf den Spender aufgesetzt wird, vorzugsweise mittels eines Gewindes aufgeschraubt wird, drückt diese Aufweitungsstruktur 62 wie im Falle von Fig. 5A bis 5F der dortige Stempel 220 die Austragstruktur 40 nach außen und bewirkt eine plastische Verformung. Vorzugsweise erfolgt auch diese Umformung unter Einfluss von Wärme. Insbesondere kann das Flüssigkeitsabgabeteil 14 zumindest partiell erwärmt sein, wenn die Schutzkappe 60 aufgesetzt wird.

## Patentansprüche

1. Verfahren zur Herstellung eines Flüssigkeitsspenders mit den folgenden Merkmalen:
a. der Flüssigkeitsspender (10) verfügt über einen Flüssigkeitsspeicher (12) sowie über eine Austragöffnung (30), durch die die Flüssigkeit in einer Austragrichtung (2A) in die Umgebung abgebbar ist, und
b. der Flüssigkeitsspender (10) verfügt über eine Gehäusebauteil (14), welches von der Austragöffnung (30) durchdrungen ist und welches eine die Austragöffnung bildende und/oder sich an die Austragöffnung (30) anschließende Austragstruktur (40) zur Beeinflussung einer Austragcharakteristik aufweist,
c. das Gehäusebauteil (14), welches von der Austragöffnung (30) durchdrungen ist, wird hergestellt, indem zunächst ein Grundkörper (14') durch Kunststoffspritzguss in einer Gussform (200) hergestellt wird und anschließend im Bereich einer vorläufigen Austragstruktur (40') des Grundkörpers (14') eine mechanische Kraftbeaufschlagung zur Verformung der vorläufigen Austragstruktur (40') und damit zur Erzeugung einer endgültigen Austragstruktur (40) erfolgt.

2. Verfahren nach Anspruch 1 mit dem folgenden weiteren Merkmal:
a. die mechanische Kraftbeaufschlagung erfolgt mittels einer Schutzkappe (60) des Flüssigkeitsspenders, die innenseitig eine sich von außen in Richtung der Austragöffnung verjüngende Aufweitungsstruktur (62) aufweist, die derart an die vorläufige Formgebung der Austragstruktur (40') des Grundkörpers (14') angepasst ist, dass dieser kelchartig aufgeweitet wird.

3. Verfahren nach Anspruch 1 oder 2 mit dem folgenden weiteren Merkmal:
a. die mechanische Kraftbeaufschlagung erfolgt mittels eines Stempels (220), vorzugsweise eines metallischen Stempels (220).

4. Verfahren nach Anspruch 3 mit dem folgenden weiteren Merkmal:
a. die mechanische Kraftbeaufschlagung erfolgt mittels eines erwärmten Stempels (220) und/oder nach Erwärmung des Gehäusebauteils (14), wobei die Temperatur des Stempels (220) bzw. das Gehäusebauteils (14) vorzugsweise mindestens der Vicat-Erweichungstemperatur des Materials des Gehäusebauteils (14) entspricht.

5. Flüssigkeitsspender (10), insbesondere in Form eines Tropfenspenders, mit den folgenden Merkmalen:
a. der Flüssigkeitsspender (10) verfügt über einen Flüssigkeitsspeicher (12) sowie über eine Austragöffnung (30), durch die die Flüssigkeit in einer Austragrichtung (2A) in die Umgebung abgebbar ist, und
b. der Flüssigkeitsspender (10) verfügt über ein Gehäusebauteil (14), welches von der Austragöffnung (30) durchdrungen ist und welches eine sich an die Austragöffnung (30) anschließende und einen Abgabepfad umgebende Austragstruktur (40) zur Beeinflussung einer Austragcharakteristik aufweist,
**gekennzeichnet durch** das folgende weitere Merkmal:,
c. die Austragstruktur (40) weist an einer Außenseite eine umlaufende Vertiefung (42) auf.

6. Flüssigkeitsspender (10) nach Anspruch 5 mit den folgenden weiteren Merkmalen:
a. der Flüssigkeitsspender (10) ist als Tropfenspender ausgebildet, und
b. die Austragstruktur (40) bildet eine Tropfenbildungsfläche (44), an der durch die Austragöffnung (30) ausgetragene Flüssigkeit zur Bildung eines Tropfens (100) anhaftet, bis sich der Tropfen (100) schwerkraftbedingt löst,
vorzugsweise mit dem folgenden zusätzlichen Merkmal:
c. die Tropfenbildungsfläche (44) weist einen Außendurchmesser von mindestens 1 mm und/oder von maximal 3 mm auf, vorzugsweise einen Außendurchmesser von mindestens 1,5 mm und/oder von maximal 2,5 mm.

7. Flüssigkeitsspender (10) nach Anspruch 5 oder 6 mit dem folgenden weiteren Merkmal:
a. die Tropfenbildungsfläche (44) ist außenseitig durch eine Abrisskante (46) begrenzt, wobei die Abrisskante (46) mit einem Krümmungsradius von mehr als 0,05 mm, insbesondere von mehr als 0,1 mm ausgebildet ist.

8. Flüssigkeitsspender (10) nach einem der Ansprüche 5 bis 7 mit dem folgenden weiteren Merkmal:
a. die Austragstruktur (40) weist außenseitig einen sich in Austragrichtung (2A) stetig aufweitenden außenseitigen Aufweitungsbereich (50) auf,
vorzugsweise mit dem folgenden zusätzlichen Merkmal:
b. ein Winkel (B) zwischen einer Oberfläche des außenseitigen Aufweitungsbereichs (50) und der Austragrichtung (2A) beträgt mehr als 20°, vorzugsweise mehr als 30°, insbesondere vorzugsweise mehr als 40°.

9. Flüssigkeitsspender (10) nach einem der Ansprüche 5 bis 8 mit dem folgenden weiteren Merkmal:
a. die Austragstruktur (40) weist innenseitig einen sich in Austragrichtung (2A) aufweitenden innenseitigen Aufweitungsbereich (52) auf,
vorzugsweise mit dem folgenden zusätzlichen Merkmal:
b. ein Winkel (B) zwischen einer Oberfläche des außenseitigen Aufweitungsbereichs (50) und der Austragrichtung (2A) ist kleiner als ein Winkel (A) zwischen einer Oberfläche des innenseiten Aufweitungsbereichs (52) und der Austragrichtung (2A).

10. Flüssigkeitsspender (10) nach einem der Ansprüche 5 bis 9 mit den folgenden weiteren Merkmalen:
a. der Flüssigkeitsspender (10) weist eine abnehmbare und wieder aufsetzbare Schutzkappe (60) auf, die die Austragöffnung (30) im aufgesetzten Zustand überdeckt, und
b. die Schutzkappe (60) verfügt über eine nach innen weisende Aufweitungsstruktur (62), die bei aufgesetzter Schutzkappe (60) an einer Innenseite der Austragstruktur (40) anliegt,
vorzugsweise mit mindestens einem der folgenden zusätzlichen Merkmale:
c. die Schutzkappe (60) ist als Schraubkappe ausgebildet, und/oder
d. die Austragstruktur (40) wird bei aufgesetzter Schutzkappe (60) von der Aufweitungsstruktur (62) unter elastischer Spannung gehalten.

11. Flüssigkeitsspender (10) nach einem der Ansprüche 5 bis 10 mit dem folgenden weiteren Merkmal:
a. die Austragöffnung (30) und/oder die Austragstruktur (40) weist eine von der Rotationsymmetrie abweichende Formgebung auf.

12. Flüssigkeitsspender (10) nach einem der Ansprüche 5 bis 11 mit dem folgenden weiteren Merkmal:
a. der Flüssigkeitsspender (10) ist als Pumpspender ausgebildet und weist eine manuell betätigbare Pumpeinrichtung (16) mit einer Pumpkammer sowie mit einem Einlassventil und einem Auslassventil auf, oder
b. der Flüssigkeitsspender (10) ist als Quetschflaschenspender ausgebildet und weist als Flüssigkeitsspender eine manuell zum Zwecke des Austrags zusammendrückbare Quetschflasche auf.

13. Flüssigkeitsspender (10) nach einem der Ansprüche 5 bis 12 mit mindestens einem der folgenden weiteren Merkmale:
a. der Flüssigkeitsspeicher (12) ist mit einer pharmazeutischen Flüssigkeit befüllt, und/oder
b. der Flüssigkeitsspeicher (12) weist ein Innenvolumen von weniger als 200 ml auf, insbesondere von weniger als 100 ml.

## Claims

1. Method for manufacturing a fluid dispenser with the following features:
a. the fluid dispenser (10) has a fluid store (12) and a delivery opening (30) through which the fluid can be discharged to the environment in a delivery direction (2A), and
b. the fluid dispenser (10) has a housing component (14) through which the continuous delivery opening (30) is made and which has a delivery structure (40) forming the delivery opening and/or adjoining the delivery opening (30), for influencing a delivery characteristic,
c. the housing component (14) having the continuous delivery opening (30) is manufactured in that firstly a base body (14') is manufactured by plastic injection moulding in a casting mould (200) and then, in the region of a provisional delivery structure (40') of the base body (14'), a mechanical force is applied to deform the provisional delivery structure (40') and hence create a definitive delivery structure (40).

2. Method according to Claim 1 with the following further feature:
a. the mechanical force is applied by means of a protective cap (60) of the fluid dispenser which, on the inside, has a widening structure (62) which tapers from the outside in the direction of the delivery opening and is adapted to the provisional form of the delivery structure (40') of the base body (14') such that this is widened in the manner of a cup.

3. Method according to Claim 1 or 2 with the following further feature:
a. the mechanical force is applied by means of a die (220), preferably a metallic die (220).

4. Method according to Claim 3 with the following further feature:
a. the mechanical force is applied by means of a heated die (220) and/or after heating of the housing component (14), wherein the temperature of the die (220) or housing component (14) preferably corresponds at least to the Vicat softening temperature of the material of the housing component (14).

5. Fluid dispenser (10), in particular in the form of a droplet dispenser, with the following features:
a. the fluid dispenser (10) has a fluid store (12) and a delivery opening (30) through which the fluid can be discharged to the environment in a delivery direction (2A), and
b. the fluid dispenser (10) has a housing component (14) through which the continuous delivery opening (30) is made and which has a delivery structure (40) adjoining the delivery opening (30) and surrounding a discharge path, for influencing a delivery characteristic,
**characterized by** the following further feature:
c. the delivery structure (40) has a circumferential groove (42) on its outside.

6. Fluid dispenser (10) according to Claim 5 with the following further features:
a. the fluid dispenser (10) is formed as a droplet dispenser, and
b. the delivery structure (40) forms a droplet formation surface (44) on which fluid discharged through the discharge opening (30) adheres to form a droplet (100) until the droplet (100) detaches under force of gravity,
preferably with the following additional feature:
c. the droplet formation surface (44) has an outer diameter of at least 1 mm and/or at most 3 mm, preferably an outer diameter of at least 1.5 mm and/or at most 2.5 mm.

7. Fluid dispenser (10) according to Claim 5 or 6 with the following further feature:
a. the droplet formation surface (44) is delimited on the outside by a breakaway edge (46), wherein the breakaway edge (46) is formed with a curvature radius of more than 0.05 mm, in particular more than 0.1 mm.

8. Fluid dispenser (10) according to any of Claims 5 to 7 with the following further feature:
a. the delivery structure (40) has on the outside an external widening region (50) which widens constantly in the delivery direction (2A),
preferably with the following additional feature:
b. an angle (B) between a surface of the external widening region (50) and the delivery direction (2A) is more than 20°, preferably more than 30°, in particular preferably more than 40°.

9. Fluid dispenser (10) according to any of Claims 5 to 8 with the following further feature:
a. the delivery structure (40) has on the inside an internal widening region (52) which widens in the delivery direction (2A),
preferably with the following further feature:
b. an angle (B) between a surface of the external widening region (50) and the delivery direction (2A) is smaller than an angle (A) between a surface of the internal widening region (52) and the delivery direction (2A).

10. Fluid dispenser (10) according to any of Claims 5 to 9 with the following further features:
a. the fluid dispenser (10) has a removable and refittable protective cap (60) which covers the delivery opening (30) when fitted, and
b. the protective cap (60) has an inwardly pointing widening structure (62) which lies against an inside of the delivery structure (40) when the protective cap (60) is fitted,
preferably with at least one of the following additional features:
c. the protective cap (60) is formed as a screw cap, and/or
d. the delivery structure (40) is held under elastic tension by the widening structure (62) when the protective cap (60) is fitted.

11. Fluid dispenser (10) according to any of Claims 5 to 10 with the following further feature:
a. the delivery opening (30) and/or the delivery structure (40) has a shape deviating from rotational symmetry.

12. Fluid dispenser (10) according to any of Claims 5 to 11 with the following further feature:
a. the fluid dispenser (10) is configured as a pump dispenser and has a manually actuatable pump device (16) with a pump chamber and with an inlet valve and an outlet valve, or
b. the fluid dispenser (10) is configured as a squeeze bottle dispenser and has as a fluid dispenser a squeeze bottle which can be compressed manually for delivery purposes.

13. Fluid dispenser (10) according to any of Claims 5 to 12 with at least one of the following further features:
a. the fluid store (12) is filled with a pharmaceutical fluid, and/or
b. the fluid store (12) has an internal volume of less than 200 ml, in particular less than 100 ml.

## Revendications

1. Procédé de fabrication d'un distributeur de liquide avec les caractéristiques suivantes :
a. le distributeur de liquide (10) dispose d'un réservoir de liquide (12) ainsi que d'une ouverture de déchargement (30), à travers laquelle le liquide peut être évacué dans l'environnement dans une direction de déchargement (2A), et
b. le distributeur de liquide (10) dispose d'un composant de boîtier (14) qui est traversé par l'ouverture de déchargement (30) et qui présente une structure de déchargement (40) formant l'ouverture de déchargement et/ou se raccordant à l'ouverture de déchargement (30) pour influencer une caractéristique de déchargement,
c. le composant de boîtier (14), qui est traversé par l'ouverture de déchargement (30), est fabriqué en fabriquant d'abord un corps de base (14') par moulage par injection de matière plastique dans un moule de coulée (200) et en appliquant ensuite, dans la zone d'une structure de déchargement provisoire (40') du corps de base (14'), une force mécanique pour déformer la structure de déchargement provisoire (40') et produire ainsi une structure de déchargement définitive (40).

2. Procédé selon la revendication 1 avec la caractéristique supplémentaire suivante :
a. l'application de la force mécanique s'effectue au moyen d'un capuchon de protection (60) du distributeur de liquide, qui présente sur le côté intérieur une structure d'élargissement (62) se rétrécissant de l'extérieur en direction de l'ouverture de déchargement, qui est adaptée à la forme provisoire de la structure de déchargement (40') du corps de base (14') de telle sorte que celle-ci s'élargit à la manière d'un calice.

3. Procédé selon la revendication 1 ou 2 avec la caractéristique supplémentaire suivante :
a. l'application de la force mécanique s'effectue au moyen d'un poinçon (220), de préférence d'un poinçon métallique (220).

4. Procédé selon la revendication 3 avec la caractéristique supplémentaire suivante :
a. l'application de la force mécanique s'effectue au moyen d'un poinçon (220) chauffé et/ou après chauffage du composant de boîtier (14), la température du poinçon (220) ou du composant de boîtier (14) correspondant de préférence au moins à la température de ramollissement de Vicat du matériau du composant de boîtier (14).

5. Distributeur de liquide (10), notamment sous la forme d'un distributeur de gouttes, avec les caractéristiques suivantes :
a. le distributeur de liquide (10) dispose d'un réservoir de liquide (12) ainsi que d'une ouverture de déchargement (30) à travers laquelle le liquide peut être évacué dans l'environnement dans une direction de déchargement (2A), et
b. le distributeur de liquide (10) dispose d'un composant de boîtier (14) qui est traversé par l'ouverture de déchargement (30) et qui présente une structure de déchargement (40) se raccordant à l'ouverture de déchargement (30) et entourant un chemin d'évacuation pour influencer une caractéristique de déchargement,
**caractérisé par** la caractéristique supplémentaire suivante :
c. la structure de déchargement (40) présente un renfoncement périphérique (42) sur un côté extérieur.

6. Distributeur de liquide (10) selon la revendication 5 avec les caractéristiques supplémentaires suivantes :
a. le distributeur de liquide (10) est réalisé sous forme de distributeur de gouttes, et
b. la structure de déchargement (40) forme une surface de formation de gouttes (44) sur laquelle le liquide déchargé par l'ouverture de déchargement (30) adhère pour former une goutte (100) jusqu'à ce que la goutte (100) se détache sous l'effet de la gravité,
de préférence avec la caractéristique supplémentaire suivante :
c. la surface de formation de gouttes (44) présente un diamètre extérieur d'au moins 1 mm et/ou d'au maximum 3 mm, de préférence un diamètre extérieur d'au moins 1,5 mm et/ou d'au maximum 2,5 mm.

7. Distributeur de liquide (10) selon la revendication 5 ou 6 avec la caractéristique supplémentaire suivante :
a. la surface de formation de gouttes (44) est délimitée du côté extérieur par un bord de rupture (46), le bord de rupture (46) étant réalisé avec un rayon de courbure supérieur à 0,05 mm, notamment supérieur à 0,1 mm.

8. Distributeur de liquide (10) selon l'une quelconque des revendications 5 à 7 avec la caractéristique supplémentaire suivante :
a. la structure de déchargement (40) présente sur le côté extérieur une zone d'élargissement extérieure (50) qui s'étend de manière continue dans la direction de déchargement (2A),
de préférence avec la caractéristique supplémentaire suivante :
b. un angle (B) entre une surface de la zone d'élargissement extérieure (50) et la direction de déchargement (2A) est supérieur à 20°, de préférence supérieur à 30°, de manière particulièrement préférée supérieur à 40°.

9. Distributeur de liquide (10) selon l'une quelconque des revendications 5 à 8, avec la caractéristique supplémentaire suivante :
a. la structure de déchargement (40) présente sur le côté intérieur une zone d'élargissement intérieure (52) s'élargissant dans la direction de déchargement (2A),
de préférence avec la caractéristique supplémentaire suivante :
b. un angle (B) entre une surface de la zone d'élargissement extérieure (50) et la direction de déchargement (2A) est inférieur à un angle (A) entre une surface de la zone d'élargissement intérieure (52) et la direction de déchargement (2A).

10. Distributeur de liquide (10) selon l'une quelconque des revendications 5 à 9, avec les caractéristiques supplémentaires suivantes :
a. le distributeur de liquide (10) présente un capuchon de protection amovible et pouvant être remis en place (60) qui recouvre l'ouverture de déchargement (30) à l'état mis en place, et
b. le capuchon de protection (60) dispose d'une structure d'élargissement (62) orientée vers l'intérieur qui, lorsque le capuchon de protection (60) est en place, s'appuie sur un côté intérieur de la structure de déchargement (40),
de préférence avec au moins l'une des caractéristiques supplémentaires suivantes :
c. le capuchon de protection (60) est réalisé sous forme de capuchon à vis, et/ou
d. la structure de déchargement (40) est maintenue sous tension élastique par la structure d'élargissement (62) lorsque le capuchon de protection (60) est en place.

11. Distributeur de liquide (10) selon l'une quelconque des revendications 5 à 10, avec la caractéristique supplémentaire suivante :
a. l'ouverture de déchargement (30) et/ou la structure de déchargement (40) présente une forme différente de la symétrie de rotation.

12. Distributeur de liquide (10) selon l'une quelconque des revendications 5 à 11 avec la caractéristique supplémentaire suivante :
a. le distributeur de liquide (10) est réalisé sous forme de distributeur à pompe et présente un dispositif de pompe (16) pouvant être actionné manuellement avec une chambre de pompe ainsi qu'avec une soupape d'entrée et une soupape de sortie, ou
b. le distributeur de liquide (10) est réalisé sous forme de distributeur à bouteille compressible et présente, en tant que distributeur de liquide, une bouteille compressible pouvant être comprimée manuellement à des fins de déchargement.

13. Distributeur de liquide (10) selon l'une quelconque des revendications 5 à 12, avec au moins l'une des caractéristiques supplémentaires suivantes :
a. le réservoir de liquide (12) est rempli d'un liquide pharmaceutique, et/ou
b. le réservoir de liquide (12) présente un volume intérieur de moins de 200 ml, notamment de moins de 100 ml.
